(19) Europäisches Patentamt / European Patent Office / Office européen des brevets

(11) **EP 2 267 422 A1**

(12) **EUROPÄISCHE PATENTANMELDUNG**

(43) Veröffentlichungstag:
**29.12.2010 Patentblatt 2010/52**

(51) Int Cl.:
*G01J 4/00* (2006.01)          *A61N 1/40* (2006.01)
*G01N 21/21* (2006.01)        *G01D 21/00* (2006.01)
*G01N 21/64* (2006.01)

(21) Anmeldenummer: **09008368.4**

(22) Anmeldetag: **26.06.2009**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**
Benannte Erstreckungsstaaten:
**AL BA RS**

(71) Anmelder:
- **Germanov, Evgeny Pavlovich**
  **Moscow 129085 (RU)**
- **Kutushov, Mikhail Vladimirovich**
  **Moscow, 125414 (RU)**
- **Grinshtein, Mark**
  **42141 Natania (IL)**
- **Shraybman, Mikhail**
  **46390 Herzlia (IL)**

(72) Erfinder:
- **Germanov, Evgeny Pavlovich**
  **Moscow 129085 (RU)**
- **Kutushov, Mikhail Vladimirovich**
  **Moscow, 125414 (RU)**
- **Grinshtein, Mark**
  **42141 Natania (IL)**
- **Shraybman, Mikhail**
  **46390 Herzlia (IL)**

(74) Vertreter: **Fleck, Hermann-Josef**
  **Klingengasse 2**
  **71665 Vaihingen/Enz (DE)**

(54) **Verfahren und Einrichtung zur Bestimmung der Malignität von biologischen Objekten**

(57)   Die Erfindung betrifft ein Verfahren zur Bestimmung des Malignisationsgrads (der Malignisierung) von Zell- und Gewebsgefügen (-Strukturen) zur Untersuchung der Präparate aus biologischen Geweben. Die Untersuchung eines beliebigen biologischen Materials (Lebewesens) wird durch die Bestimmung des Grades der Anisotropie und der Isotropie der Gewebsgefüge (-strukturen) aufgrund eines ausgeprägten Grades der Symmetrie und Dissymetrie von superschwachen elektromagnetischen Schwingungen (Informationssignale) vorgenommen. Diese Schwingungen (Informationssignale) entstehen bei dem zu untersuchenden biologischen Material im Kreis der Einrichtung für den vegetativen Resonanztest. Die Signale werden durch einen Polarisator durchgelassen. Dabei werden die Richtung und der Grad ihrer Polarisation bei der für das Bindegewebe eigenen Resonanzfrequenz als Indikator des Grades der Malignität verwendet. Bei der Auswertung wird berücksichtigt, dass ein maligne Gewebe durch ein symmetrisches (isotropes) Signal und ein Normalgewebe durch ein dissymetrisches (anisotropes) Signal gekennzeichnet ist.

   Die Einrichtung zur Durchführung des Verfahrens weist eine Auflagefläche (eine Schale) für das zu untersuchende Material auf. Diese Auflagefläche ist unterhalb eines unteren festen Polarisator angeordnet. Über der Auflagefläche und dem festen Polarisator sind zwei Polarisatoren angeordnet, zwischen denen sich ein Kristall befindet. Dabei ist einer der Polarisatoren fixiert, der andere ist an einem beweglichen Ring mit einer Teilung (Eichung) darauf befestigt. Über dem oberen beweglichen Polarisator befindet sich ein Behälter (eine Schale), welcher mittels eines Kabels mit der Aufnahmestelle für MT (medikamentöses Testen) des Gerätes für VRT (vegetativen Resonanztest) verbunden ist. Dabei können die Auflagefläche zum Testen und der Behälter aus einem beliebigen Werkstoff ausgebildet sein.

   Die Einrichtung zur Bestimmung des Grades der (Symmetrie) Isotropie des biologischen Materials weist einen Polarisator auf. Der Polarisator ist in Form von zwei Kristallen gebildet. Dabei ist der eine Kristall über einem unteren unbeweglichen Polarisator angeordnet, der zweite Kristall befindet sich unter dem oberen beweglichen Polarisator und bewegt sich zusammen mit dem letzteren.

   Dabei werden:
die Zuverlässigkeit der Daten bei der Bestimmung (des Grades) der Malignität von Zell- und Gewebsstrukturen (-gefügen) und der Bestimmung des Grades der Isotropie (Symmetrie) von biologischem Material (Objekt) erhöht, die Verfahrenstechnik vereinfacht, die für die Untersuchung in Anspruch genommene Zeit verkürzt und die Funktionalität für die Diagnosestellung von verschiedenen Pathologien und für die Bestimmung von aussichtsreichen Richtungen der Behandlung ergänzt.

Fig. 2

**Beschreibung**

Die Erfindung betrifft ein Verfahren und eine Einrichtung zur Bestimmung der Malignität von Zell- und Gewebestrukturen (-gefügen) und des Grads der Isotropie (Symmetrie) von biologischem Material (Objekt) nach dem Oberbegriff des Anspruch 1.

Die Erfindung kann in der Medizintechnik eingesetzt werden und umfasst einen Polarisator, der mit einer Testeinheit für VRT (vegetativer Resonanztest) und/oder einer Einheit für BRT (Bioresonanztherapie) vereinigt ist.

Zur Bestimmung der Malignität von Geweben und Zellen werden zurzeit Instrumentaluntersuchungsverfahren angewendet:

1. Radioisotopendiagnostik. In der onkologischen Praxis wird eine Radioisotopenszintigraphie der Organe vorgenommen, falls ein Verdacht auf Tumoraffektion primär oder metastasisch besteht. Das Verfahren der Kontakt-Beta-Radiometrie wird bei der Diagnostik von Hautmelanomen, bei oberflächlichen Mammatumoren und bei Paget-Krankheit eingesetzt.

2. Radionuklid-Verfahren. Die Radionuklid-Verfahren sind sehr aussichtsreich (dabei ist die Generation von tumorspezifischen markierten Antikörpern gemeint). Zurzeit wird die Szintigraphie von Skelett, Gehirn und Lungen zur Diagnose und die Szintigraphie von Nieren und Leber zur Beurteilung ihres funktionellen Zustands angewendet.

3. Die Radioimmununtersuchung beruht auf einer Analyse des moniklonalen Antikörpergehalts in den zu untersuchenden Geweben. Sie ermöglicht es, viele Tumorarten in den Frühstufen der Prozessentwicklung zu erkennen, wenn die Tumore noch kleine Abmessungen aufweisen. Die Tests zur Erkennung von Antikörpern, welche für eine bestimmte Tumorart spezifisch sind, ermöglichen es, das Problem der zu niedrigen Kontrastauflösung zwischen den Geweben mit ähnlicher Dichte sehr wirksam zu lösen. Den üblichen Röntgenuntersuchungen ist eine solche geringe Kontrastauflösung eigen.

4. Magnetresonanztomographie (MRT) ist ein Bildgebungsverfahren, bei dem die Bilder durch ein kernmagnetisches Resonanzsignal induziert werden. Der grundsätzliche Unterschied zwischen MRT und CT besteht darin, dass die zu messende Größe bei MRT die Magnetisierungsintensität von bestimmten innerhalb des gesonderten Volumenteils vorhandenen Kernen ist. Bei CT dagegen wird der Absorptionsfaktor der Röntgenstrahlung durch unterschiedliche biologische Gewebe gemessen. Die klinische Anwendung des MRT-Verfahrens besteht in der Untersuchung der räumlichen Verteilung der Kerne von Wasserstoff, Phosphor und manchen anderen Elementen im Körper des Menschen. Die bei der MRT zu erfassende Hauptgröße ist die Reaktion (Antwort) der Magnetkerne auf die Einwirkung des Magnetwechselfeldes. Diese Reaktion hängt von der Kerndichte und anderen Kennwerten ab, welche für jeden einzelnen Körperbereich spezifisch sind.
Die Vorteile der MRT:

Das Verfahren ermöglicht es, einen außerordentlich hohen Kontrast der Gewebe zu erreichen. Im Rahmen einer Untersuchung bekommt man die Darstellungen in allen anatomischen Ansichten. Die dynamischen Abläufe werden im Zusammenhang mit der Bewegung von biologischen Flüssigkeiten (Blut, Liquor, Harn, Galle) untersucht. Dank der Anwendung von Kontrastmitteln wird das peritumorale Ödem von dem eigentlichen Tumor mit hohem Genauigkeitsgrad unterschieden. Die Mängel von MRT bestehen in den ziemlich hohen Kosten im Zusammenhang mit der Untersuchung sowie in der Unmöglichkeit ihrer Anwendung, wenn die Patienten ferromagnetische Implantate haben.

5. Kernresonanz-Spektroskopie in vivo. Die Vervollkommnung der Forschungstechnik erweiterte den Umfang der mit NMR lösbaren Aufgaben. Sie ermöglichte es, immer kompliziertere Objekte zu untersuchen, insbesondere die Struktur von biologischen Molekülen und ihre Funktionen im Organismus auf Zellenniveau. Die in vivo erfasste biochemische Information kennzeichnet das Niveau der Energieversorgung der Zellen und insbesondere von Metabolismus innerhalb des gesonderten Abschnitts eines beliebigen biologischen Gewebes. Sie ermöglicht es dem Onkologen, sehr wertvolle Angaben über das Vorhandensein und die Art des Tumors, über seine Malignitätsstufe und über den Erhaltungsgrad der Organe und Systeme zu erhalten. Diese Angaben ergänzen die Daten der MRT-Untersuchung. Eine besondere Bedeutung wird der metabolischen Information beigemessen. Diese Information wird im Rahmen der dynamischen Untersuchung der Patienten mit onkologischen Pathologien erfasst. Sie ermöglicht es, die Behandlungseffektivität stufenweise zu beurteilen. Dabei werden die optimale Heilmittel- bzw. Bestrahlungsdosis gewählt und die sofortigen bzw. Fernreaktionen auf die durchgeführte Therapie erfasst. Unter den zahlreichen klinischen Anwendungen des Verfahrens kommen die meisten auf die Untersuchungen der Patienten mit Gehirntumoren zu.

6. Positronen-Emissionstomographie (PET) ist ein äußerst wirksames Verfahren für klinische Untersuchungen der Patienten mit onkologischen Pathologien. Seine weite Verbreitung in den letzten zehn Jahren hängt vor allem mit der Entwicklung und der technischen Verbesserung unterschiedlicher Geräte zusammen, welche für die Untersuchung des Gesamtkörpers bestimmt sind. Die PET ermöglicht es, einmalige Informationen über die metabolische Aktivität der Tumore sowie über die Metabolismusänderungen zu erhalten, welche auf die durchgeführte Therapie zurückzuführen sind. Je nach Geschwindigkeit und Intensität der Speicherung von isotopmarkierten Metaboliten oder Sonderheilmitteln kann über die biologischen Besonderheiten des Geschwulstgewebes im Vergleich zum Intaktgewebe geurteilt werden. Es wird auch möglich, die Behandlungseffektivität auszuwerten und eine Prognose für den weiteren Prozessverlauf zu machen. Das ist in der Krebsforschung besonders wichtig.

7. Thermographie. Dieses Verfahren umfasst die Erzeugung eines Körperbildes des Menschen anhand eines Sondergerätes, welches gegen die Infrarotstrahlung (Wärmeausstrahlung) empfindlich ist. Auf diesem Bild werden Körperbereiche mit unterschiedlicher Hauttemperatur mit verschiedenen Farben markiert. Die thermographische Mammauntersuchung ermöglicht es, die Krebsaffektionen bei 80 - 87 % der untersuchten Personen zu diagnostisieren. Die Kombination der Thermographie mit einer Röntgenuntersuchung erhöht die Genauigkeit der Diagnose mit dieser Lokalisation bis zu 99 %. Die Thermographie ist bei der Diagnose von Schilddrüsenkrebs und Hautmelanom sehr effektiv.

Neben den Instrumentalverfahren gibt es auch interventionelle und nichtinterventionelle Untersuchungsverfahren.

1. Bei der Biopsie handelt es sich um die Exzision oder das Abkneifen eines kleinen Tumorstücks oder eines evtl. verdächtigen Gewebeteilchens zwecks nachfolgender histologischer Untersuchung. Bei der totalen Biopsie wird der gesamte Tumorknoten oder der metastasenverdächtige Lymphknoten vollständig entfernt. Wird zu Untersuchungszwecken nur ein Teil des Tumors oder des tumorverdächtigen Gewebes exzidiert, so wird von Inzisionsbiopsie gesprochen. Die Biopsie wird vielfach in poliklinischen Einrichtungen bei endoskopischen Untersuchungen des Mastdarmes und des Dickdarmes, des Gebärmutterhalses und anderer Organe angewendet. Wird das Material für die histologische Untersuchung mittels einer speziellen oder üblichen Injektionsnadel entnommen, so wird diese Biopsie Punktionsbiopsie genannt. In onkologischen Bereichen und in den Abteilungen der poliklinischen Einrichtungen wird die Punktionsbiopsie normalerweise mit einer feinen Injektionsnadel vorgenommen. Gleichzeitig werden aus dem mittels Aspiration gewonnenen Material 1-2 und mehr zytologische Abstriche entnommen, die ins klinische Labor zum Zytologen weitergeleitet werden.

2. Markerdiagnostik. Es gibt drei Gruppen von Geschwulstwachstummarkern, die mit Proteinen, RNS und DNS assoziiert sind. Die proteinassoziierten Marker können in gewöhnlichen Blutproben gemäß den routinemäßigen biochemischen und immunchemischen Verfahren erkannt werden. Unter solchen Markern können das prostataspezifische Antigen (PSA) beim Vorsteherdrüsenkrebs, das CA 125 beim Eierstockkrebs, das karzinoembryonale Antigen (KEA) bei Karzinomen der Verdauungstraktorgane, menschliches Ghoriongonadotropin bei Trophoblasttumoren und Alpha-Fetoprotein bei hepatozellulärem Krebs und Embryonalkarzinomen genannt werden. Die Erkennung dieser Proteine ist für die Prognosestellung und das Krankheitsmonitoring für die frühzeitige Bestimmung von Tumormetastasen und -rezidiven sehr wichtig. Jedoch ist die Anwendung dieser Marker beschränkt, weil sie für die jeweiligen Tumore bzw. Tumorarten nicht spezifisch genug sind. Ihr Vorhandensein im Blut weist des Öfteren auf den bereits verbreiteten Prozess hin. Die Grundlage für die Diagnose von malignen Geschwülsten nach dem Biopsie-Material ist die Auswertung von den nach üblichen Verfahren gefärbten histologischen und zytologischen Präparaten. Gleichzeitig erfordert die Anwendung von Protokollbehandlung, die Histogenese der Tumore unbedingt zu präzisieren. Das betrifft besonders die undifferenzierten Tumore. Die regionären und Fernmetastasen müssen so früh wie möglich festgestellt werden. Es ist jedoch auf der Basis der Untersuchung mit Einsatz von histologischen Routinemethoden nicht immer möglich, den Charakter der Neubildungen und die Differenzierungsstufe der die Neubildung bildenden Zellen festzustellen. Es ist manchmal sehr schwer, die Krebszellen in den Lymphknoten und im Knochenmark und Exsudaten aus Serosenhöhlen in den Frühstufen der Metastasenbildung zu entdecken. Diese Schwierigkeiten können evtl. anhand von Molekularbiologieverfahren überwunden werden. Diese Verfahren umfassen Immunhistochemie und Immunzytochemie, welche auf Erkenntnissen der Biotechnologie und Daten über die molekularen Besonderheiten der Tumorzellen beruhen. Zu diesem Zweck wurden die immunhistochemischen Verfahren unter Einsatz von moniklonalem Antikörpergehalt (MKAG) an die so genannten tumorassoziierten Antigene verwendet. Die tumorassoziierten Antigene umfassen onkofetale, gewebespezifische, organspezifische Antigene, Proteine der Zwischenfilamente und die Antigenreihe von Blutgruppen und Gewebeverträglichkeit, Fermente, Hormone und die mit Onkogenen kodierten Proteine. Um diese an den Oberflächenmembranen und im Zellenzytoplasma zu erkennen, wurden hochspezifische und empfindliche Verfahren zur immunoenzymometrischen histochemischen Analyse (PAP, APAAP, ABC, EnVision, LSAB und a.m.) entwickelt. Diese Verfahren können bei der Auswertung von Kryostat- und Paraffinschnitten und Abdrücken verwendet werden, welche bei der Exzisionsbiopsie der Tumore, der Abstriche aus Feinnadelpunktaten der Lymphknoten und des Knochenmarks, der Zellenelemente der Rückenmarkflüssigkeit und Exsudaten aus Serosenhöhlen erhalten wurden.

Es gibt noch keine genauen Tests zur Feststellung des Krankheitsstadiums vor der Operation. Es gibt auch keine sicheren und zugänglichen Kriterien zur Erkennung von Kranken mit einem hohen Metastasenentwicklungsrisiko nach der Entfernung des Primärtumors. Die Feststellung der Verbreitung des Tumorprozesses vor dem Entstehen seiner klinischen Merkmale ist von großer Bedeutung, besonders in Bezug auf die richtige Wahl der Behandlungstaktik. Die Onkologen lassen sich mit dem am häufigsten angewandten Verfahren zur Feststellung solcher Proteine, wie CA 19-9, CA 242, CA 125, CA 50, PSA, KEA, Alpha-Fetoprotein, Ghoriongonadotropin und andere im Blutserum der Kranken, nicht zufrieden stellen, da der Gehalt an diesen Proteinen auch bei Nichtgeschwulst-Krankheiten und bei Leberfunktionsstörungen erhöht sein kann. Es sei auch bemerkt, dass diese Markerproteine sich meistens erst bei einem entwickelten Tumorprozess erkennen lassen. Somit können sie zur frühzeitigen Feststellung der Metastasenbildung oder zur Einschätzung seines Risikogrades kaum nutzbar sein.

Dank den Erkenntnissen der immunozyto- und immunhistochemischen sowie der molekularbiologischen Technik können heutzutage die so genannten Mikrometastasen (bzw. okkulte Metastasen) in Lymphknoten, Abspülungen aus der Pleurahöhle und Bauchhöhle, im Blut und im Knochenmark festgestellt werden. Die in der Fachliteratur erwähnten Angaben weisen darauf hin, dass die Anwendung von MKAG an die Proteine des Zytoskeletts, an eine Reihe von gewebe- und organspezifischen und tumorassoziierten Antigenen es ermöglicht, die Tumorzellen in Lymphknoten und anderen biologischen Objekten mit einem hohen Genauigkeitsgrad zu entdecken. Von größtem Interesse sind dabei die Zytokeratine. Zytokeratine sind Proteine, die unentbehrliche Bestandteile des Zytoskeletts von normalen und in bösartig umgewandelte Epithelzellen sind. Die Zytokeratine werden ausschließlich durch die Geschwülste mit Epithelherkunft repräsentiert. Die Anwendung von MKAG an die Zytokeratine ermöglicht es, die einzelnen Krebszellen unter den sie umgebenden 100000 bis 1000000 Nichtgeschwulstzellen des Lymphknotens oder Knochenmarks zu identifizieren. Somit können Zytokeratine als zuverlässige Marker der Zellen mit Epithelherkunft betrachtet werden. (Artikel aus der Fachzeitschrift "DOCTOR" Heft 4, 2003. Internet)

Die Wissenschaftler aus der Purdue-Universität (Lafayette, Indiana) und aus dem Lawrence-Nationallabor (Berkeley, California) haben ein neues Verfahren entwickelt. Dieses Verfahren ermöglicht es, die Normalspaltzellen gegenüber den bösartigen Zellen zu differenzieren. Das wird durch die Feststellung der Lokalisation des spezifischen Kernproteins anhand eines Fluoreszenzfarbstoffes erreicht. Die Forscher haben im Rahmen ihrer Arbeit die Verteilung von Kernprotein mit einem Mitoseapparat (NuMA) erforscht je nach Umfang des Mammazellenkerns, abhängig davon, ob dieser bösartige, nicht vollständig gereifte oder ganz normal funktionierende Zellen enthält. Dafür wurde am NuMA-spezifischen Antikörper ein Fluoreszenzmolekül befestigt. Das Glimmen dieses Moleküls ermöglichte es, die Proteinansammlungen in bestimmten Kernbereichen zu erfassen. Das von diesen Wissenschaftlern entwickelte System nahm den Vergleich der Informationen über die Proteinlokalisation mit den Kenndaten jedes Typs der zu untersuchenden Zellen automatisch vor. Im Endeffekt wurde eine 3D-Karte erstellt. Anhand dieser Karte konnte die Verteilung von NuMA in den Kernen der zu untersuchenden Zellen studiert werden. Die Verfasser dieses Verfahrens sind der Meinung, dass es nach einer bestimmten Zeit nicht nur zum Nachweisen von Malignität der Zelle, sondern auch zur genauen Feststellung der Krebsart, zur Bestimmung der Wahrscheinlichkeit seiner Metastasenentwicklung sowie zur Auswahl der am besten passenden und effektvollen Behandlungsmethode angewendet werden kann. Rein theoretisch kann eine ähnliche 3D-Karte für ein beliebiges Kernprotein erstellt werden, welches in den Zellenmalignisierungsabläufen eingesetzt wird. Das sollte die Aufgabe der richtigen Diagnosestellung sowie der Erhöhung der Behandlungseffektivität von onkologischen Erkrankungen wesentlich vereinfachen.
(Internetzeitung "Commercial Biotechnology", abgefasst nach den Materialien von Purdue University)

Jedoch weisen alle oben erwähnten Verfahren explizite und verdeckte Mängel auf. Zu den Hauptmängeln zählen: Teuerung und Kompliziertheit der Verfahren und die Notwendigkeit, in die körperlichen Gewebe einzudringen. Darüber hinaus weisen alle hier aufgezählten Verfahren keine 100%-ge Diagnosesicherheit auf.

Es ist Aufgabe der Erfindung, ein einfaches und sicheres Verfahren zur Frühdiagnose der malignen Zustände zu schaffen, das sogar die Motivation der Gewebe und Zellen in Bezug auf Malignisation bestimmen sowie die morphologische Art des Tumors feststellen kann.

Diese Aufgabe wird durch die Merkmale des Anspruchs 1 gelöst.

Der gesamte lebendige Körper und alle seine Systeme stellen eine Quelle von schwachen elektromagnetischen (physiologischen oder harmonischen) Schwingungen innerhalb eines breiten Frequenzspektrums dar. Die Krankheitserreger haben oft auch ihre eigenen Frequenzen. Im Falle der Erkrankung werden im Organismus neue Quellen von elektromagnetischen Schwingungen - die so genannten "pathologischen" oder "disharmonischen" Schwingungen - generiert, welche das physiologische Gleichgewicht stören.

Die Bioresonanztherapie (BRT) ist eine neue Richtung in der Entwicklung der Heil- und Vorbeugungsmedizin. Die Geräte für die Bioresonanztherapie haben mit den Schwingungen des menschlichen Körpers nach dem Induktionsprinzip zu tun. Die patienteneigenen Schwingungen und Signale weisen einen elektromagnetischen Charakter auf.

Deswegen ist ihre Übertragung über Drähte möglich. Das wurde bereits experimentell bewiesen (Schimmel, 1978). Ihre Ableitung vom Körper des Patienten wird anhand von Elektroden über ein Kabel ausgeführt, welches sie an das Gerät hinleitet. Diese Schwingungen werden durch eine Präzisionseinrichtung geleitet, welche diese Schwingungen in polungsmäßige Rückschwingungen umwandelt. Danach kommen die umgewandelten Schwingungen in Form verstärkter Daten über ein zweites Kabel zum Patienten zurück. Das bedingt die Löschung bzw. die Abnahme der Menge von pathologischen Daten. Für Behandlungszwecke wird in den Bioresonanztherapiegeräten das so genannte "Schwingungsmodell des Patienten" benutzt. In diesem Fall wird das Isotherapieprinzip "aequalia aequalibus curentur" eingesetzt. Jedoch werden als therapeutisches Agens nicht die Substanzen und Materialien aus dem Organismus des Patienten eingesetzt (Autonosode, wie es in der "biochemischen" Isotherapie der Fall ist), sondern seine elektromagnetischen Schwingungen. D. h., es kann über "biophysikalische" Isotherapie gesprochen werden. Die Bioresonanztherapie funktioniert in der biophysikalischen Ebene und stellt somit eine elektromagnetische Resonanztherapie dar. Das grundsätzliche Prinzip der Bioresonanztherapie ist die Inversion: Die Schwingungen werden an der Hand des Patienten aufgenommen, kommen am Eingang des Geräts an, werden um 180° invertiert, verstärkt und vom Ausgang des Geräts aus an die andere Hand des Patienten weitergeleitet. Dabei werden die pathologischen Schwingungen gedämpft (beseitigt), und die Normalschwingungen, d.h. die physiologischen Schwingungen werden nur etwas abgeschwächt, was einen Trainingsfaktor darstellt.

Eine Reihe von Geräten für die Bioresonanztherapie wird in Deutschland hergestellt. Dazu zählen "BICOM", "RITEC 2000" und andere. Diese Geräte beinhalten eine Einrichtung, die als Separator bezeichnet wird. Der Separator ermöglicht es, physiologische und pathologische Schwingungen zu trennen. Physiologische Schwingungen werden unverändert an den Patienten zurückgeführt. Pathologische Schwingungen werden dagegen invertiert, verstärkt und kommen ebenfalls für die nachfolgende Therapie zurück. In der Regel ist der Separator eine ziemlich teuere und sehr komplizierte Einrichtung. Außerdem sind die damit erfassten klinischen Ergebnisse qualitativ nicht besser als die Ergebnisse, welche ohne Separator erzielt worden sind. (http://www.npl-rez.ru/litra/brt.html].

Die Grundlage für den vegetativen Resonanztest (VRT) bildet eine festgestellte Tatsache, und zwar:

Alle biologischen Lebensformen haben ein einmaliges, d. h. nur jeder Lebensform eigenes spezifisches Frequenzspektrum von elektromagnetischen Schwingungen.
Der menschliche Frequenzbereich reicht von 1520 bis 9460 kHz. Pathogene Organismen (Pilze, Viren, Helminthen, Zecken) haben einen Bereich von 77 bis 900 kHz.
Somit werden alle Frequenzkennlinien von Parasiten, Pilzen, Viren erkannt und erfasst, welche nur diesen Lebensarten allein eigen sind.

Die VRT-Diagnose ermöglicht es, den aktuellen Gesamtzustand des Patienten festzustellen, d. h.:

1 den Schädigungsgrad des Immunsystems festzustellen;
2 die Funktionskorrektur des Immunsystems vorzunehmen;
3 die Viren-, Bakterien- und mykotische Belastung des Organismus und ihre Lokalisierung in den Organen zu bestimmen;
4 Toxine aus dem Körper abzuleiten;
5 die Organe mit unterschiedlichen Störungen zu entdecken;
6 die Effektivität und die Verträglichkeit in Bezug auf Heilmittel zu bestimmen;
7 die gesamte Allergiebetroffenheit (allergische Belastung) und Allergene, die Abnahme und die Normalisierung der körperlichen Reaktionen auf Antigen, Allergen zu bestimmen,
8 die Voronkologie und Onkologie festzustellen;
9 die optimale Hilfstherapie zu wählen;
10 einen Vitamin-, Enzyme-, Hormon- und Spurenelementemangel festzustellen;
11 die zystischen Prozesse zu erkennen;
12 die geistig-seelische Beanspruchung zu beurteilen,
13 die Anpassungsfähigkeit des ZNS zu normalisieren und zu erhöhen;
14 das biologische Alter zu bestimmen, mit dessen Hilfe schwere degenerative Erkrankungen, Onkologie, Stresszustände festgestellt werden können;
15 die Anpassungsreserven des Körpers, sowohl aktuelle Optimalreserven als auch höchstmögliche Reserven, eines konkreten Menschen einzuschätzen;
16 den Grad von Signifikanz von psychologischen Problemen bei der Erschöpfung der Anpassungsreserven und folglich bei der Entwicklung von Pathologie des jeweiligen Patienten zu beurteilen.

Darüber hinaus werden die einzelnen Probleme in den Organen und Systemen bereits im Stadium von funktionellen Änderungen festgestellt, d.h., wenn noch keine physische Verletzung des Organs vorliegt. Dadurch kann die Entwicklung einer Krankheit vermieden werden. Dieses Verfahren wird zurzeit als ein ziemlich präzises Verfahren anerkannt.

Durch zahlreiche Forschungen wurde nachgewiesen, dass der Hauptinformationsträger sowohl innerhalb des biologischen Objekts als auch zwischen den einzelnen biologischen Objekten, darunter auch unter Menschen, die elektromagnetische Strahlung (EMS) ist. Der eigentliche Vorgang der Informationsübertragung ist ein energetischer, räumlicher und Zeitablauf.

Die Forschungen haben gezeigt, dass die Raum-Zeit-Struktur der Außenmakrowelt durch eine sich ununterbrochen wiederholende Sequenz von Einwirkungen zu einem chemischen Kontinuum der molekularen Mikrowelt von Lebewesen transformiert wurde. Sie trug zur Umwandlung chemischer Strukturen in Funktionsstrukturen bei. Die lebendige und nichtlebendige Materie bestehen aus den gleichen Elementen des Periodensystems. Bei der Abgrenzung der lebendigen und nichtlebendigen Materie ist die Gefügebeschaffenheit ein wesentlicher Faktor, jedoch gilt dies nur dann, wenn das jeweilige Gefüge über eine bestimmte Funktion verfügt. Der Übergang vom Leben zum Tod mit der Aufrechterhaltung des vorhandenen Gefüges ist jedoch mit der Beendigung von den das Leben bestimmenden Funktionen nur für die lebendige Materie möglich. Die allgemeinen Eigenschaften der lebendigen Form umfassen die Fähigkeit zur Selbstreproduktion. Die kennzeichnende Haupteigenschaft besteht jedoch darin, dass jedes Lebewesen ein individuelles informationsverfügendes Gefüge (Informations- und Steuerstruktur) aufweist, da eine Selbstreproduktion ohne Übertragung der Erbinformation und der Entwicklungsprogramme unmöglich ist.

Technische Lösung des Problems

Gemäß der Theorie von M.V. Kutushov lässt die Diskrepanz zwischen dem bestehenden Informationsumfang und dem Zellenspeichervermögen (Zellengedächtnisvermögen) das Vorhandensein sowie die Funktion einzigartiger Informationscluster im Lebewesen in Form von Informationskristallstrukturen annehmen. Diese Informationskristallstrukturen lassen sich noch lange nach dem Tod aus dem Organismus oder aus dem entfernten Gewebe oder Organ ablesen. Spezifische Schwingungen der Informationscluster haben offensichtlich keine Bedeutung. Nachdem die Substanzen mit einer bestimmten geometrischen Konfiguration im lebendigen Gewebe gewesen sind, prägen sie sich die Besonderheiten dieses Gewebes für eine unbestimmte Zeitdauer ein. Die aggressiven Agens wie Formalin sind ebenfalls nicht imstande, die Informationen in solchen Gefügen zu "löschen". Diese Gefüge weisen eine nanokristallinische Herkunft und einen nanokristallinischen Aufbau auf. Eigentlich ist die Struktur dieser Cluster sowie ihr Geometrieaufbau die Lagerstelle für die gesamte im lebendigen Organismus gesammelte Information. Zu solchen Informationsclustern gehören auch die DNS-Moleküle und die Proteinstrukturen.

Bekanntlich weisen die DNS-Moleküle eine positive doppelte Lichtbrechung auf, d.h., sie polarisieren das Licht nach rechts. Die Proteine dagegen weisen eine negative doppelte Lichtbrechung auf, d. h., sie polarisieren das Licht nach links. Diese Erscheinung wird als Dissymmetrie bezeichnet. Bei einem Lebewesen sind alle Normalgewebe und alle ihre Gefüge asymmetrisch (anisotrop), während die Krebsgewebe und ihre Strukturen symmetrisch (isotrop) sind. Diese Regel bleibt auch nach dem Tod des Organismus oder des aus dem Organismus entfernten Gewebes erhalten, weil die holographische Matrix dieser Strukturen erhalten bleibt. Das Normalprotein und die DNS-Moleküle resonieren mit einem bestimmten Frequenzspektrum. Ihre holographische Matrix resoniert mit denselben Frequenzen. Auch nach dem Tod und nach der Entfernung von Geweben aus dem Organismus fahren sie fort, die Hintergrundstrahlung zu generieren. Je nach der Geometrie der Gewebegefüge wird die Potenz und die Polarisationsrichtung unterschiedlich sein. Gemäß der Theorie von M.V. Kutushov gehen sogar die nur geringfügig malignisierten Gewebe in die höheren Syngonien (Würfel, Sphäre) über. Das legt den Grad der Anisotropie und der Isotropie fest. Die höheren Singonien haben keine Symmetrieachsen, deswegen sind sie isotrop. Folglich sollte von den malignisierten biologischen Objekten und entfernten Geweben das unpolarisierte (isotrope, symmetrische) Signal erhalten werden. Dementsprechend sollte von gesunden biologischen Objekten und von gesunden entfernten Geweben das polarisierte (anisotrope, asymmetrische) Signal erhalten werden.

(M.V. Kutushov «Krebs ist heilbar», herausgegeben von V. Sekachev, 2005. S. 86-87).

Die technische Aufgabe der Gruppe von Erfindungen, welche einen einheitlichen Erfindungsgedanken aufweisen, besteht darin:

- ein wirksames Verfahren und eine wirksame Einrichtung zur Bestimmung der Malignität von Zell- und Gewebsstrukturen (-gefügen), und
- eine Einrichtung zur Bestimmung des Grades der Isotropie (Symmetrie) von biologischem Material (Objekt) zu schaffen sowie
- das Inventar von wirksamen Verfahren und Einrichtungen zur Bestimmung des Grades der Malignität von Zell- und Gewebsstrukturen (-gefügen) und von Einrichtungen zur Bestimmung des Grades der Isotropie (Symmetrie) von biologischem Material (Objekt) zu ergänzen.
  Die gestellte Aufgabe kann dadurch gelöst werden, dass

- die Zuverlässigkeit der Daten bei der Bestimmung (des Grades) der Malignität von Zell- und Gewebsstrukturen (-gefügen) und der Bestimmung des Grades der Isotropie (Symmetrie) von biologischem Material (Objekt) erhöht wird,
- die Verfahrenstechnik vereinfacht wird,
- die für die Untersuchung in Anspruch genommene Zeit verkürzt wird,
- die Funktionalität für die Diagnosestellung von verschiedenen Pathologien und für die Bestimmung von aussichtsreichen Richtungen der Behandlung ergänzt wird.

Das Wesen der Erfindung hinsichtlich der Bestimmung der Malignität von Zell- und Gewebsstrukturen (-gefügen) besteht darin, dass ein Verfahren zur Untersuchung der Präparate aus biologischem Gewebe vorgesehen ist. Das Verfahren ist dadurch gekennzeichnet, dass die Untersuchung eines beliebigen biologischen Materials (Lebewesens) durch die Bestimmung des Grades der Anisotropie und Isotropie der Gewebsgefüge (-strukturen) aufgrund eines ausgeprägten Grades der Symmetrie und Dissymmetrie von superschwachen elektromagnetischen Schwingungen (Informationssignale) verwendet wird. Diese Schwingungen (Informationssignale) entstehen bei dem zu untersuchenden biologischen Material im Kreis der Einrichtung für den vegetativen Resonanztest. Sie werden durch einen Polarisator durchgelassen. Dabei werden die Richtung und der Grad ihrer Polarisation bei der für das Bindegewebe eigenen Resonanzfrequenz als Indikator des Grades der Malignität angesehen. Bei der Auswertung wird berücksichtigt, dass das maligne Gewebe durch ein symmetrisches (isotropes) Signal und das Normalgewebe durch ein dissymetrisches (anisotropes) Signal gekennzeichnet ist.

Der Index der Symmetrie (Isotropie) und der Dissymmetrie (Anisotropie) wird nach folgendem Verhältnis berechnet:

$$I_{CT} = C \cdot (D : L)$$

dabei sind

I     der Symmetrieindex des Informationssignals
C     ein normales Signal des Bindegewebes
D     ein Signalpegel mit rechter Polarisation
L     ein Signalpegel mit linker Polarisation

In Sonderfällen der Ausführung des Verfahrens wird das zu untersuchende Material durch

- ein histologisches Präparat,
- Vollblut, Blutplasma oder eine andere biologische Flüssigkeit aus der Gruppe: Urin, Speichel, Sperma, Tropfen und / oder
- ein Stück von einem Makropräparat bzw. seinem Abdruck auf einem beliebigen Material

auf einem Objektglas repräsentiert.

In anderen Sonderfällen wird als das zu untersuchende Biomaterial ein biologisches Subjekt (Mensch, Tier und Ähnliches) verwendet.

In weiteren Sonderfällen der Ausführung wird als das zu untersuchende Biomaterial eine Information genommen, die diesem Material entnommen und in einem Informationsspeicher gesichert wurde (Kapsel, homöopathisches Granulat, Alkohol, Wasser usw.).

In weiteren Sonderfällen der Ausführung wird als das zu untersuchende Material ein beliebiges lebendiges oder nicht lebendiges Material oder eine lebendige oder nicht lebendige Substanz verwendet.

Es wird bevorzugt, dass der Grad der Malignität des Gewebes mittels Präparate aus der Gruppe Farbstoffe, flüssige Kristalle u. a. Werkstoffen bestimmt wird. Diese Werkstoffe sollen Polarisations- bzw. Oszillationseigenschaften aufweisen. Die Behandlung der festgestellten onkologischen und/ oder somatischen Erkrankungen wird mittels des Polarisators durchgeführt. Der Polarisator wird dabei in den Kreis der Bioresonanztherapie (BRT) eingefügt.

Das Wesen der Erfindung hinsichtlich der Einrichtung zur Ausführung des oben dargestellten Verfahrens besteht darin, dass die Einrichtung eine Auflagefläche (eine Schale) aufweist. Diese Auflagefläche ist unterhalb eines unteren festen Polarisator angeordnet. Über der Auflagefläche und dem festen Polarisator sind zwei Polarisatoren angeordnet, zwischen denen sich ein Kristall befindet. Dabei ist einer der Polarisatoren fixiert, der andere ist an einem beweglichen Ring mit einer Teilung (Eichung) darauf befestigt. Über dem oberen beweglichen Polarisator befindet sich ein Behälter (eine Schale), welcher mittels Kabel mit einer Aufnahmestelle für MT (medikamentöses Testen) des Gerätes für VRT (vegetativen Resonanztest) verbunden ist. Dabei können die Auflagefläche zum Testen und der Behälter aus einem beliebigen Werkstoff ausgebildet sein.

Die Einrichtung zur Bestimmung des Grades der (Symmetrie) Isotropie des biologischen Materials besteht darin, dass der Polarisator in dieser Einrichtung in Form von zwei Kristallen dargestellt ist. Dabei ist der eine Kristall über dem unteren unbeweglichen Polarisator angeordnet, der zweite Kristall befindet sich unter dem oberen beweglichen Polarisator und bewegt sich zusammen mit dem letzteren.

In den Sonderfällen der Ausführung der Einrichtung kann der Polarisator aus magnetischem oder nicht magnetischem Material ausgebildet sein. Zwischen den zwei gekreuzten (und / oder parallelen) Polarisatoren ist ein beliebiger flüssiger oder fester Kristall (z. B. Quarz oder Islandspat) beweglich oder unbeweglich angeordnet. Die Achsen dieses Kristalls verlaufen parallel (und/oder normal) zum Vektor des Strahlungsstromes.

Bei der Ausführung des Verfahrens mit Hilfe der oben beschriebenen Einrichtungen unterscheiden sich die superschwachen elektromagnetischen Schwingungen (Informationssignale) dem Symmetriegrad nach. Diese Schwingungen (Informationssignale) kommen von dem Normal- und Krebsgewebe mit unterschiedlichen Frequenzen zurück (oder werden davon reflektiert). Die isotropen (Krebs-) Gewebe funktionieren in einem verzerrten Modus wie "Rauschen". Dadurch ist das reflektierte zurückkommende Signal isotrop. Die Isotropie zeigt sich durch die symmetrische Antwort, welche in der vorgeschlagenen Einrichtung empfangen wird. Dabei geben die Krebsgewebe nur ein symmetrisches Signal. Die Normalgewebe senden eine dissymmetrische Antwort, welche je nach morphologischem, physiologischem und Funktionszustand variiert.

Funktionsweise des vorgeschlagenen Signalpolarisators:

Das Signal wird von einem beliebigen biologischen Objekt (Gewebe, Blut, gistologisches Präparat usw.) oder vom biologischen Feld des Patienten aufgenommen und durch einen Analysator (Polarisator) gelassen.

Der Polarisator besteht aus Polarisationsfiltern, welche sich in Bezug auf einander und den Kristall drehen. Das Signal vom Geräteausgang beim Standpunkt "0" des Polarisators und bei der Stellung des Polarisators um 5° nach rechts und nach links vom "0"-Punkt fließt in die diagnostische Anlage zum vegetativen Resonanztest ein.

Die Ergebnisse werden nach den Kennwerten einer Resonanzskala des Bindegewebes abgelesen.

Der Test auf das Vorhandensein eines bösartigen Prozesses gilt als positiv, wenn im "0"-Punkt der Polarisatorstellung niedrige Skala-Werte und bei der Wendung des Polarisators um den gleichen Winkelwert nach rechts und nach links vom "0"-Punkt symmetrische Werte abgelesen werden. Das negative Ergebnis der Prüfung auf das Vorhandensein eines malignen Tumors liegt dann vor, wenn Hochwerte im "0"-Punkt der Resonanzskala des Bindegewebes sowie unterschiedliche Skala-Werte, d. h. die Dissymmetrie, bei der Wendung des Polarisators um den gleichen Winkelwert nach rechts und nach links vom "0"-Punkt abgelesen werden. Die histologische Verifikation des Tumors, seine Lokalisation, der Grad der Prozessmalignität, der Zustand der Gegenkrebs-Resistenz usw. werden nach den Skalen des vegetativen Resonanztests festgestellt.

Die vorgeschlagene Einrichtung stellt im Grunde genommen eine Art von Separator dar, welcher bei der Bioresonanzprüfung eingesetzt wird.

Der Stand der Technik gegenüber der vorgeschlagenen Einrichtung ist die so genannte chromatische Polarisation. Ausgerechnet bei der Beobachtung der Darstellung des Objektes im Polarisationsmikroskop haben die gleich gefärbten Teile des Objektes ungefähr die gleiche optische Stärke. Die weiße Polarisationsfärbung ist eine Erscheinung, welche das "Anfärben" des Ausgangslichtbündels umfasst. Das Lichtbündel passiert zwei gekreuzte Polarisatoren und den dazwischen angeordneten einachsigen Kristall. Die Achse des Kristalls verläuft normal zur Lichtverbreitungsrichtung und bildet mit den Achsen der Polarisatoren einen Winkel ungleich 90° aus.

Der gesamte Effekt beruht völlig auf der Zustandsänderung der Strahlungspolarisation bei Verbreitung der Strahlung im einachsigen Kristall.

Bei der Null-Anisotropie der Brechungszahl Dn oder bei der Nullstärke L des einachsigen Mediums zwischen den Polarisatoren (mit anderen Worten, bei ihrer Abwesenheit oder Isotropie) ist der Durchlässigkeitsgrad dieses Systems strikt gleich Null, T = 0. Bei der Anisotropie des Phasenanlaufs im Kristall ungleich Null $j = 2pDnL/l$. Das Transmissionsspektrum weist sehr schnelle Schwingungen bei ausreichend großen (einige Dutzenden von $\mu m$) Werten von d auf. Wenn die d-Werte ca. 1 $\mu m$ betragen, ist die Spektralabhängigkeit des Durchlassvorgangs ziemlich gleichmäßig und hat nur ein bis zwei Max.-Spitzen im sichtbaren Spektrum. Diese Spitzen entsprechen der Bedingung $j = p/2 + pm$. In diesem Fall nimmt das durchfallende Licht eine sichtbare "Färbung" an. Das wird als chromatische Polarisation der Strahlung bezeichnet.

Die technische Ausführung des Effekts besteht im Folgenden: Das von der Lichtquelle ausgehende Parallellichtbündel passiert 2 gekreuzte Polarisatoren (Trenner). Dabei ist kein passiertes Bündel_vorhanden. Danach wird zwischen den Polarisatoren der Kristall angeordnet. Das passierte Bündel wird gefärbt. Indem der Strahlungseinfallwinkel (der auf eine Platte einfällt) geändert wird, wird eine gleichmäßige Änderung der "Färbung" des passierten Lichtbündels erreicht. Die optische Polarisation wird zur Qualitäts-Express-Analyse in der Polarisationsmikroskopie der räumlichinhomogenen anisotropen Objekte (kleiner unregelmäßig geformter Naturkristallite, dünner Probestücke von ausgerichteten Flüssigkristallen und a. m.) eingesetzt. (Fundstelle: http://www.effects.ru)

Quellennachweis:

1. Sivukhin D.V. Allgemeine Physik. Optik. Оптика.) - M.: Nauka, 1985.

2. Landsberg G.S. Optik. (Ландсберг Г.С. Оптика) - M.: Nauka, 1976.

3. Physik. Großes Lexikon. (Физика. Большой энциклопедический словарь) - M.: Bolschaja Rossijskaya Enzyklopedia, 1999. - S.90, 460.)

Dieser Effekt wurde in der von uns vorgeschlagenen Einrichtung angewendet, um die Aufgabe der Unterscheidungslehre für somatische und onkologische Erkrankungen zu lösen. In unserer Einrichtung wurde anstatt eines Lichtbündels ein elektromagnetisches (Informations-) Signal von dem zu untersuchenden Objekt der geregelten zirkularen Polarisation und der Trennung in die linke und rechte Polarisation ausgenutzt.

Der andere nächste Prototyp ist die Sondendiagnostik des Objektes mittels des Parallellichtbündels oder mittels des divergenten (konischen) Lichtbündels. Im ersten Fall werden Parallelprojektionen und im zweiten Fall kegelförmige Projektionen gebildet.

Eventuelle weitere Sondierungsmöglichkeiten:

1 Wendung des Bündels der Sondierungsstrahlung in Bezug auf das unbewegliche Objekt;
2 Drehung (Neigung) des Objekts in Bezug auf das unbewegliche Bündel der Sondierungsstrahlung.

Die Diagramme mit der Wendung des Bündels in Bezug auf das unbewegliche Objekt unterscheiden sich nach der Laufbahn, welche vom Sondierungsbündel in Bezug auf das Objekt oder das Gebiet (Netz) mit den darauf erfassten Projektionen unterschieden werden. Es sind folgende Ansätze bekannt:

1 das außeraxiale sich drehende Punktdiaphragma in der Ebene der Öffnungsblende des Mikroobjektivs. Die Sondierungslaufbahn ist ein Kreis;
2 eindimensionale Verlagerung der Punktquelle in der Ebene der Offnungsblende des Kondensators und die Sicherstellung einer Schrägbeleuchtung. Die Sondierungslaufbahn ist eine Gerade (1 D);
3 der Einsatz von der Mikrospiegelmatrix DMD (Digital Micromirror Devices). Die Mikrospiegel werden in die Offnungsblende des Mikroobjektivs gesetzt, um die Schrägbeleuchtung zu generieren. Dieses Schema kann nur bei kleinen Objekten benutzt werden. Die Sondierungslaufbahn ist eine beliebige zweidimensionale Laufbahn (2D);
4 zweidimensionale Verlagerung der Punktquelle in der Ebene der Offnungsblende des Mikroobjektivs und die Herstellung einer Schrägbeleuchtung. Die Sondierungslaufbahn ist eine beliebige zweidimensionale Laufbahn (2D).

Unter diesen Schemata werden diejenigen am meisten bevorzugt, welche die zweidimensionale Laufbahn sicherstellen. Alle Ansätze weisen einen gemeinsamen Mangel auf und zwar: Der Sondierungswinkel des Objektes ist durch die numerische Apertur des Mikroobjektivs begrenzt und überschreitet nicht 90e. (Patent der RF Nr. 2140661 (Russland) - Verfahren der konfokalen 3D-Scanningmikroskopie und das konfokale tomographische Scanningmikroskop. Levin G.G., Vishnyakov G.N., Bulygin F.V. -angemeldet am 19.03.99).

Jedoch ist auch dieses Verfahren nicht imstande, das erwartete Ergebnis zu erreichen, um das elektromagnetische (Informations-) Signal in die rechte und linke Polarisation aufzuteilen.

Diese Aufgabe wird anhand der vorgeschlagenen Einrichtung (Polarisator) des elektromagnetischen (Informations-) Signals gelöst.

Die Haupteinheit der Einrichtung ist eine Testeinheit zur Prüfung der Präparate nach dem VRT-Verfahren. Die Haupteinheit zur Trennung der Polarisation des Informationssignals ist der vorgeschlagene Polarisator (Polaroid). Der Polarisator (ähnlich wie bei einem Polarisationsfilter - Polaroid) lässt die entlang der ausgesonderten Richtung polarisierte elektromagnetische Komponente des (Informations-) Signals ungehindert durch und nimmt das Informationssignal mit senkrechter Polarisation völlig auf.

Die Einrichtung zur Feststellung der Symmetrie (Isotropie) von malignen Geweben besteht aus einem unbeweglichen Teil in Form eines Ringes und einem beweglichen Teil in Form einer Scheibe mit einer Teilung (Eichung) darauf. Auf dem unbeweglichen Teil sind ein oder zwei Polarisatoren für das linearpolarisierte Licht befestigt. Als Polarisator kann eine Folie verwendet werden. Die Folie kann aus folgenden Materialien bestehen: Turmalin, Zelluloseazetatschicht mit einer großen Menge von Kleinkristallen von Herapathit (jodhaltige Verbindung von schwefelsaurem Chinin), Jod-polyvinylfolien mit den gleich ausgerichteten Polymerketten. Es können auch die Stapel (die Pakete) von feinen Platten von isotropen Stoffen eingesetzt werden, in denen die "überflüssige" Komponente an den Plattengrenzen gedämpft wird.

Polarisatoren können mechanisch, ferritisch (elektromagnetisch) und impuls-ferritisch sein. Sie können aus beliebigem Material ausgebildet sein, welches das Vermögen besitzt elektromagnetische Wellen (Informationssignale) von beliebiger Länge bzw. Frequenz zu polarisieren.

Zwischen den Polarisatoren wird ein Kristall mit einer bestimmten Art von Kristallgitter angeordnet. Der Kristall kann aus folgendem Material ausgeführt werden: Glas, beliebiger Kristall, Islandspat, Material mit dem dispergierten Fastkristall oder ein anderes Material, welches das Vermögen besitzt, die elektromagnetischen Wellen, das Licht und das Informationssignal zu polarisieren.

In den Kreis der Bioresonanztherapie (BRT) ist ein Polarisator eingefügt. Dieser Polarisator empfängt Signale, welche der Kranke ausstrahlt. Das Signal wird "umgewendet" (invertiert) und "gefärbt". Dabei bekommt es die nötige (physiologisch gesunde) Frequenzmodulation und gelangt in den Organismus zurück. Nachdem das Signal in die Zell- und Gewebsgefüge (-strukturen) gelangt ist, fangen diese an, im Resonanzmodus den normalen Metabolismus und die normale Struktur wiederherzustellen.

Das Informationssignal geht über die gekreuzten Polarisatoren und/oder den Kristall am Ausgang der Einrichtung und zeigt den Symmetriegrad des zu untersuchenden Materials an. In der vereinfachten Form ist die Polarisationseinheit durch nur einen einfachen Polarisator oder einen festen oder sich drehenden einfachen Kristall vertreten. Obwohl es sich normalerweise um einen Lichtstrom handelt, welcher in einem solchem System polarisiert wird, kann nach den zahlreichen Testergebnissen geurteilt werden, dass das elektromagnetische (Informations-) Signal, welches sowohl von unlebendigen als auch von lebendigen Objekten gesendet wird, ebenfalls über Polarisationsfähigkeit verfügt. Sowohl unlebendige als auch lebendige Krebsgefüge geben eine symmetrische Antwort (Isotropie) und lebendige und gesunde Strukturen geben eine dissymmetrische (asymmetrische) Antwort (Anisotropie). Gemäß den erfassten Ergebnissen sind die Polarisation des Informationssignals und die übliche optische und elektromagnetische Polarisation identisch. Folglich ist eine solche Eigenschaft der Anisotropie (Polarisation) oder der Isotropie des zu untersuchenden Materials kennzeichnend (inhärent).

Das angemeldete Verfahren verwendet zur Feststellung der Anisotropie (Dissymetrie) und der Isotropie (Symmetrie) des zu untersuchenden Materials einen Polarisator. Zur Bestimmung der Malignität der biologischen Gefüge können sowohl lebendige Objekte als auch histologische Präparate benutzt werden.

Die Erfindung wird anhand von in den Zeichnungen dargestellten Ausführungsbeispielen näher erläutert. Es zeigen:

Fig. 1    ein allgemeines Schaltbild einer Einrichtung zum vegetativen Reso- nanztest (VRT) mit einem Polarisator,

Fig. 2    eine erste (vereinfachte) Ausführungsform eines Polarisators,

Fig. 3    eine Ausführungsform der Einrichtung mit Färbung des elektromagne- tischen (Informations-)Signals in den gekreuzten Polarisatoren und einen Kristall. Unter den gekreuzten Polarisatoren werden Polarisato- ren verstanden, welche mit einem Winkelwert von 0 bis 90 Grad durch Drehen gewendet werden und

Fig. 4    eine Ausführungsform mit einem unbeweglichen (beweglichen) Kris- tall (oder einem Behälter mit einem flüssigen Kristall) und einer dreh- baren (nicht drehbaren) Unterlage für das zu untersuchende Präparat.

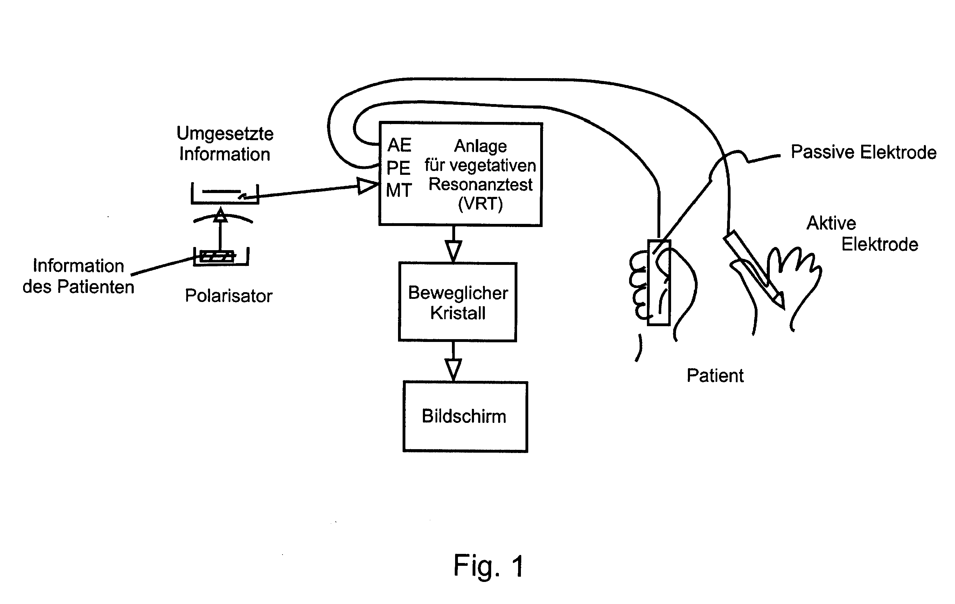

Es bezeichnen in Figur 1:

1 - einen Polarisator,

2 - ein Gerät für vegetativen Resonanztest (VRT),

3 - ein aus Software und Gerätetechnik bestehendes System mit Anschlussstellen: AE - aktive Elektrode, PE - passive Elektrode, MT - Aufnahmestelle für medikamentöses Testen, über welche ins Gerät für vegetativen Resonanztest die umgewandelte Information kommt, welche in Potenz-Einheiten des Bindegewebes gemäß einer 100-Punkte-Skala, ausgedruckt ist,

4 - einen Bildschirm,

5 - eine aktive Elektrode und

6 - eine passive Elektrode.

Die Elektroden 5 und 6 befinden sich in den Händen des Patienten.

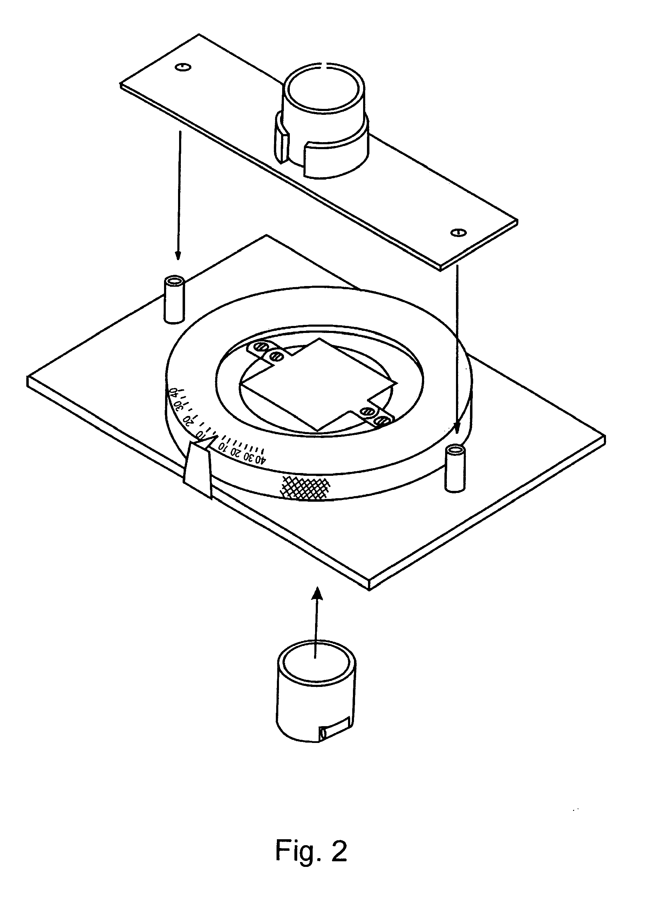

Es bezeichnen in Figur 2:

7 - eine Auflage für das zu untersuchende Material (der untere Zylinder mit dem nach oben zeigenden Zeiger),

8 - ein beweglicher Ring mit einer Eichung und dem unbeweglichen Polarisator für polarisiertes Licht und

9 - eine Auflagefläche für die Platte zum Empfang von Informationen (der Zylinder oben).

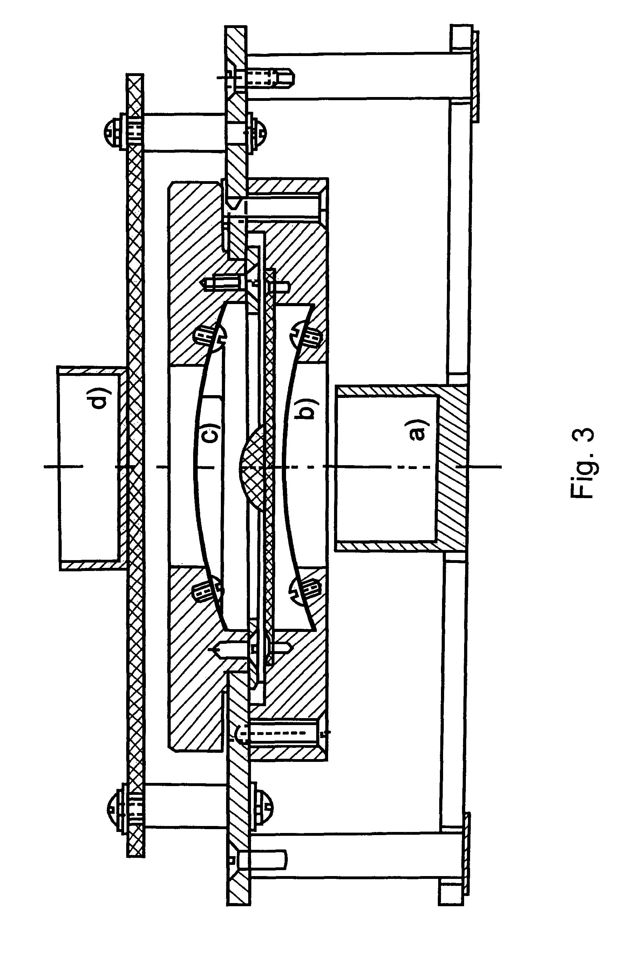

Es bezeichnen in Figur 3:

10 - eine Auflagefläche für das zu untersuchende Material (oder Eingabe durch den Patienten),

11 - einen unbeweglichen Ring mit einem daran befestigten unbeweglichen Polarisator,

12 - einen beweglichen Ring mit einer Eichung und einem daran befestigten unbeweglichen Polarisator,

13 - eine Auflagefläche für die Elektrode zum Empfang von Informationen (darin ist eine Kupferplatte mit einem abführenden Leiter eingebaut),

14 - einen zwischen den Polarisatoren (b und c) angeordneten unbeweglichen Kristall (in Form einer Hemisphäre dargestellt).

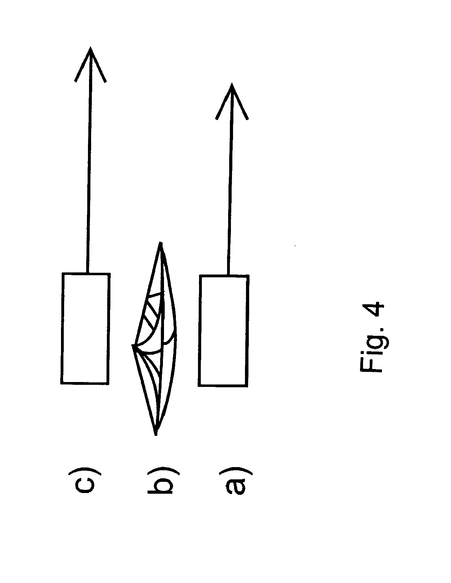

Es bezeichnen in Figur 4:

15 - eine Auflage für die Präparate (bzw. eine Ableitung zum Tester oder zum biologischen Objekt, Organismus),

16 - einen Kristall (z.B. der Behälter mit einem Kristall) und

17 - eine passive Elektrode als Ausgang zur Anlage für einen vegetativen Resonanztest (VRT).

Die Untersuchungen eines biologischen Objektes (eines histologischen Präparats, einem Stück des Gewebes, eines Bioptats oder ihrer Abdrücke auf einer Gaze) werden nach folgender Methodik durchgeführt:

Das Informationsfeld, das vom biologischen Objekt empfangen wird, wird auf einen Informationsträger mittels eines Magnetinduktors oder auf eine andere geläufige Weise aufgenommen. Der Informationsträger wird auf die Auflage unter den Polarisator gesetzt. Das Signal geht durch den Polarisator hindurch, kommt an die Abnahmevorrichtung und wird über das Kabel und die Aufnahmestelle für MT (medikamentöses Testen) an das Gerät für den vegetativen Resonanztest (VRT) übertragen.

Das Verfahren zum vegetativen Resonanztest beruht auf dem Resonanzeffekt. Der letztere entsteht während der Messung des elektrokutanen Widerstandes. Dabei wird ein superschwacher Gleichstrom (etwa 300 NM) zugeführt. An das System wird ein zusätzliches Informationssignal gesendet, welches dem im Organismus des Patienten ähnlich ist.

Der elektrokutane Widerstand eines Menschen ist von dem Zustand des vegetativen Nervensystems abhängig, d.h. von dem Tonusverhältnis seines sympathischen und parasympathischen Teils. Ein erhöhter Hautwiderstand (Senkung des Meßpegels gemäß der Skala des Gerätes für den vegetativen Resonanztest) zeigt eine Erhöhung des Tonus des parasympathischen Teils bzw. eine Senkung des Tonus des sympathischen Teils des vegetativen Nervensystems an.

Wird dem Organismus eine unterschiedliche Menge von "Ampullen" des Informationspräparats Epiphyse eingegeben, welches den Tonus des umherschweifenden Nervs erhöht, so erhöht sich die Empfindlichkeit des parasympathischen Teils des vegetativen Nervensystems und dementsprechend auch der elektrokutane Widerstand. Daher muss vor der Durchführung der Untersuchung die Anzahl der Ampullen von Epiphyse ausgewählt werden, welche gleich n-1 ist und eine Erhöhung der Empfindlichkeit sicherstellt. D.h. soviel, dass die Hinzugabe noch einer Ampulle eine starke Erhöhung des elektrokutanen Widerstands verursacht. Dabei spricht man von dem Zeigerabschlag-Phänomen (Abschlag des Zeigers auf der Geräteskala).

Die Intensität des empfangenen Signals, das durch den Polarisator durchgelassen wird, wird nach den Kennwerten einer Resonanzskala des Bindegewebes abgelesen. Das Bindegewebe gibt einen biofunktionalen Zustand der Organe und der Systeme wieder.

Das vorliegende Gerät ist ein zusätzliches Modul für ein Gerät für einen vegetativen Resonanztest (VRT).

Dieses Modul besteht aus:

- zwei Polarisatoren, welche ein System bilden. Dabei dreht sich einer der Polarisatoren hinsichtlich des anderen,
- einer unter dem unteren Polarisator angeordneten Auflage. Auf diese Auflage wird eine Schale mit dem Informationsträger gestellt,
- einer Abnahmevorrichtung mit einer Aufnahmestelle für das Verbindungskabel, das an den MT-Anschluss des Geräts für den vegetativen Resonanztest (VRT) angeschlossen ist,
- eine Skala mit Gradteilung, welche die Winkelwerte der Drehung des Polarisators anzeigt.

Das Gerät ist so justiert, dass bei dem Zeiger der Skala auf 90° das Informationspräparat STK D2000 (0-Potenz des Bindegewebes) nach der vollen Resonanzskala des Bindegewebes resonieren wird. Die Skala ist mit einem □-Zeichen markiert. Das Informationspräparat STK D2000 ist als Last auf der unteren Auflage unterhalb des unteren Polarisators (anstatt des zu untersuchenden Präparats) aufgebracht. Bei der Drehung des Polarisators werden höhere Potenzen resonieren. Bei dem Zeiger der Skala auf 0° wird die Potenz des Bindegewebes D2000 resonieren.

Jedes gefertigte Gerät hat seine eigenen charakteristischen Merkmale. Aus diesem Grund muss der Arzt vor dem unmittelbaren Vorgehen eine Tabelle aufstellen, welche die Potenzen der vollen Resonanzskala des Bindegewebes des medikamentösen Auswählers (Selektionsgeräts) und die entsprechenden Winkelwerte der Drehungen des Polarisators in 5-Grad-Schritten angibt. Aufgrund der ermittelten Daten ergibt sich ein Winkel der Wendung des Drehung, welcher für die Untersuchung optimal ist und die maximale Dissymmetrie angibt. Dabei muss die Spanne$\Delta$ zwischen den zu untersuchenden resonierenden Potenzen des Bindegewebes über dem Kennwert 10 bei dem gleichen Winkelwert der Drehung des Polarisators nach links und nach rechts berücksichtigt werden. Dies ist für das Normalgewebe kennzeichnend.

Beispiel der Winkelwert-Potenz-Tabelle

| Winkelwert der Wendung des Polarisators | Nach rechts (STK-Potenz) | Nach links (STK-Potenz) | $\Delta$ |
|---|---|---|---|
| 90 Grad | □ □ | □ □ | -- |
| 80 | 5 | 8 | -3 |
| 75 | 12 | 15 | -3 |
| 70 | 19 | 22 | -3 |
| 65 | 31 | 40 | -9 |
| 60 | 49 | 59 | -10 |
| 55 | 66 | 80 | -14 |
| 50 | 85 | 89 | -4 |
| 45 | 86 | 89 | -3 |
| 40 | 90 | 88 | +2 |
| 35 | 92 | 86 | +6 |
| 30 | 95 | 83 | +12 |
| 25 | 97 | 79 | +18 |
| 20 | 98 | 75 | +23 |
| 15 | 99 | 82 | +17 |
| 10 | 99 | 88 | +11 |
| 5 | 100 | 95 | +5 |
| 0 | 100 | 100 | -- |

Demgemäß ergibt sich der maximale Dissymetrie-Kennwert dieses Geräts bei einem Winkelwert der Drehung des Polarisators mit 20° ($\Delta$ = 23). Zugleich ist es möglich, den Test mit den Winkelwerten der Drehung von 25°, 15°, 55°, 30°, 10° und 60° durchzuführen. Um den Charakter der Pathologie bei der vollen Symmetrie oder bei der nicht ausgeprägten Dissymmetrie feststellen, können zusätzliche Untersuchungen anhand von vegetativem Resonanztest (VRT) vorgenommen werden. Es können festgestellt werden:

- der Zustand des psycho-vegetativen, endokrinen und Immunsystems,

- die allgemeinen Gegenkrebs-Resistenz des Organismus,
- das Testen von Psorinum, Carcinosinum, voronkologischen Prozessen,
- der onkologische Test des Krebsproteins und des genormten Proteins,
- die Filtration der Organopräparate (in Potenz $\Delta = 3$) durch die ermittelte Potenz des Krebsproteins,
- der Zustand der Organopräparate nach der Skala des Bindegewebes,
- die Gegenkrebs-Resistenz und das Malignitätspotential der Organopräparate,
- falls mit dem Krebsprotein mehrere Organopräparate getestet werden, wird darunter das primär betroffene Organ gewählt,
- das von dem onkologisch Kranken empfangene und invertiert aufgenommene Signal wird über den Polarisator untersucht. Dabei ändert das Signal seine STK-Kennwerte nicht, d.h. es bleiben niedrige Potenzen des Bindegewebes und der Symmetrie erhalten. Dabei wird das von einem Patienten ohne onkologische Erkrankung empfangene Signal invertiert und es erhöhen sich die Potenz des Bindegewebes und der Symmetrie-Wert in bedeutendem Maße,
- falls die onkologisch Kranken und die Kranken die Voronkologie OF-Test-Präparate genommen haben, dann erhöht sich die Potenz des Bindegewebes ebenfalls. Es wird Dissymetrie festgestellt.
- bei der Stellung auf 90° ist STK gleich 3. Bei der Drehung des Polarisators nach rechts ist STK gleich 5, bei der Drehung des Polarisators nach links ist STK gleich 5. Es liegt Symmetrie vor. Durch das Krebsprotein wird das Organpräparat des Dickdarmes getestet. Die Nosode resoniert - es wurde ein Adenokarzinom entdeckt. Auf der Platte für MT wird ein OF-Test gesetzt: die Kennwerte nach der STK-Skala sind jetzt 23/ 35/ 50 (anstatt 3/ 5/ 5). Das zeugt von einem onkologischen Prozess, die Behandlung ist mit Hilfe dieser Präparate möglich.
- bei einem weiteren Beispiel: ist STK gleich 2, STK über den Polarisator ist gleich 3/ 6/ 9. Die Kennwerte nach der Zustand-Skala sind 2/ 1, also ein mittlerer Zustand der Gegenkrebs-Resistenz. Das Krebsprotein ist gleich $\Delta = 100$, das genormte Protein - $\Delta = 30$. Voronkologischer Prozess -2. Über das C-Protein wird das Organpräparat des Kehlkopfs getestet, $\Delta = 3$. Das auf einen Träger aufgenommene Organpräparat des Kehlkopfs $\Delta = 3$ wird über den Polarisator untersucht, es werden nach der STK-Skala Kennwerte 0/ 2/ 3 ermittelt. Bei der Beladung mit dem OF-Test-Präparat ergeben sich Kennwerte 9\ 36\ 52. Das Krebsprotein wird nicht getestet, das genormte Protein ist gleich □. Die Gegenkrebs-Resistenz ist sehr hoch.

Die unten angeführten Beispiele dienen zum Nachweis der Objektivität des Verfahrens und der Einrichtungen.

Beispiel 1.

Zur Bestimmung der Malignität eines entfernten Tumors (histologisches Material) wurden histologische Präparate genommen, welche nach genormten Verfahren angefertigt wurden.

U. Mk.: Gistologisches Bild von Gliosarkom (Ratte). Das Material wurde den Ratten mit eingeimpftem malignem Gehirntumor (Gliosarkom L9) entnommen.

Der Polarisator wurde um 5° nach rechts und dann auch um 5° nach links gesetzt. Auf der Bildschirmskala wird ein Symmetriegrad (Isotropie) von 4/4 angezeigt. Der Polarisationsgrad (Anisotropie) ist gleich 0. D.h., es liegt vollständige Isotropie vor.

Beispiel 2.

Das histologische Präparat wurde aus einem Dünndarmstück nach einer Resektion im Zusammenhang mit der Crohns-Krankheit gefertigt.

Der Polarisator wurde um 5° nach rechts und dann auch um 5° nach links gesetzt. Das Signal betrug im 1. Fall 16 und im zweiten Fall 23. Es liegt eindeutig Dissymmetrie vor.

Beispiel 3.

Patientin mit Beschwerden durch eine Verhärtung im Bereich der rechten Brustdrüse und durch einen vergrößerten Achsellymphknoten. Es wurde eine Punktionsbiopsie des Tumors sowie eine Lymphknotenresektion mit nachfolgender morphologischer und histologischer Untersuchung vorgenommen. Vor der Operation wurde nach dem Verfahren der nicht invasiven Polarimetrie (der vegetative Resonanztest (VRT) + Polarisator) folgende Diagnose gestellt: Duktaler Brustkrebs mit Metastasen im Achsellymphknoten.

Makropräparat: Der Achsellymphknoten wurde auf die Auflage für das zu untersuchende Material gesetzt.

Der Polarisator wurde um 5° nach rechts und dann auch um 5° nach links aufgestellt. Das Signal betrug im ersten Fall 4 und im zweiten Fall 4. Es liegt offensichtliche Symmetrie vor. Die Anisotropie ist gleich 0. Diagnose: Metastatischer Lymphknoten beim duktalen Brustkrebs.

Makropräparat: Brustfragment mit abgeändertem Gewebe wurde auf die Auflage für das zu untersuchende Material gesetzt.

Der Polarisator wurde um 5° nach rechts und dann auch um 5° nach links aufgestellt. Das Signal betrug im ersten Fall 4 und im zweiten Fall 4. Es liegt offensichtliche Symmetrie vor. Die Anisotropie ist gleich 0. Diagnose: Milchgangkarzinom beim Brustkrebs. Die Kontrolluntersuchung der Morphologie und des histologischen Präparats nach allgemein anerkanntem Verfahren bestätigte die Diagnose, die mittels Polarisators gestellt wurde. Es wurde eine Totalmammaabtragung mit Exstirpation von 3 Achsellymphknoten vorgenommen.

Beispiel 4.

Der Tropfen aus dem Prüfglas mit Blutplasma von einem Patienten mit Magenkrebs wurde auf einen Steriltupfer aufgetragen und dann unter den Polarisator gesetzt. Der Polarisator wurde um 5° nach rechts und dann auch um 5° nach links aufgestellt. Auf der Bildschirmskala wird der Symmetriegrad (Isotropie) von 6/6 angezeigt. Der Polarisationsgrad (Anisotropie) ist gleich 0. D.h., es liegt vollständige Isotropie vor.

Beispiel 5.

Nach der Dickdarmresektion wurde die Untersuchung des Abdruckes des vermutlichen Dickdarmkrebses vorgenommen. Der Polarisator wurde um 5° nach rechts und dann auch um 5° nach links aufgestellt. Auf der Bildschirmskala wird der Symmetriegrad (Isotropie) von 6/6 angezeigt. Der Polarisationsgrad (Anisotropie) ist gleich 0. D.h., es liegt vollständige Isotropie vor.

Beispiel 6.

Der Bluttropfen eines Patienten mit unspezifischer ulzeröser Kolitis wurde unter den Polarisator gesetzt. Der Polarisator wurde um 5° nach rechts und dann auch um 5° nach links aufgestellt.
Auf der Bildschirmskala wird ein Symmetriegrad (Isotropie) von 5/5 angezeigt. Der Polarisationsgrad (Anisotropie) ist gleich 0. D.h., es liegt vollständige Isotropie vor. Aufgrund dieses Testes kann auch ein Dickdarmkrebs diagnostiziert werden.

Beispiel 7.

Patient mit Diagnose Hochdruckkrankheit. Beschwerden über zu hohen Blutdruck und Kopfschmerzen. Es wurde eine Untersuchung auf das Vorhandensein von Krebsprotein und den Grad (Stufe) von Dissymmetrie vorgenommen. Der Polarisator wurde um 5° nach rechts und dann auch um 5° nach links aufgestellt. Auf der Bildschirmskala wird ein Symmetriegrad (Isotropie) von 6/6 angezeigt. Der Polarisationsgrad (Anisotropie) ist gleich 0. D.h., es liegt 100% Isotropie vor. Aufgrund dieses Tests kann angenommen werden, dass bei dem Patienten ein_ Anfangsstadium von Krebs ohne klinische Erscheinungen vorliegt. Während der Untersuchung anhand von CT wurde festgestellt, dass an der Spitze der rechten Lunge des Patienten ein Herd mit den Abmessungen von 2 x 3 cm vorhanden ist. Es wurde die Resektion der Spitze der rechten Lunge durchgeführt. Die histologische Untersuchung zeigte ein kleinzelliges Lungenkarzinom an.

Beispiel 8.

Kranker mit chronischer Lungen- und Herzinsuffizienz, 64 J.
Es wurde ein onkologischer Test nach dem genormten Nosode (Carcinosinum) durchgeführt. Der Test ergab keine Krebspathologie. Es wurde eine Prüfung auf Symmetrie der Organopräparate durchgeführt. Im Bereich der Leber wurde ein Pathologiekern festgestellt. Dieser Pathologiekern lässt sich durch das Vorhandensein von großer Isotropie erkennen. D.h., dass die Intensitätswerte des Informationssignals eine symmetrische Antwort anstreben. Die Kontrast-CT stellte das Vorhandensein von kleinen Bildungen mit Sekundärgenesis im linken Leberlappen fest. Später wurde klargestellt, dass der Primärherd sich in der aufsteigenden Abteilung des Dickdarms in Form eines 2 x 2 cm großen Polyps befand. Der Polyp wurde exzidiert. An dieser Stelle wurden dann die Zellen von Adenokarzinom entdeckt. Der Patient machte eine DST-Behandlung im Laufe von 2 Monaten durch. Die Kontrolldiagnostik mittels Polarisation zeigte, dass keine Metastasen mehr vorhanden waren. Bei der Kontrast-CT wurden ebenfalls keine Neubildungen mehr erkannt.

Beispiel 9.

Der Patient mit chronischer Pneumonie wurde einer Untersuchung auf dem Polarisator mit gekreuzten Polarisatoren und dem einachsigen Kristall unterzogen. Bei der Wendung um 5° nach rechts wurde die Antwort 4 und bei der Wendung um 5° nach links die Antwort 4 zurückgegeben. Die Dissymmetrie ist nicht ausgeprägt. Der obere Polarisator ist um 90° zum unteren Polarisator verlagert. Bei dieser Aufstellung zeigt die Polarisation 5 nach rechts und 7 nach links. Es liegt eine mäßige Dissymmetrie vor, was eine chronische Pneumonie mit einer Neigung zur Malignisation bedeutet.

Beispiel 10.

Das DST-Präparat wurde auf das Objektglas aufgetragen und auf die Auflage für die Prüfung gesetzt.

Der Polarisator wurde in die Stellung 5° nach rechts gebracht. Das Signal vom Präparat ergab die Übereinstimmung mit Adenokarzinomen von Prostata, Mamma, Magen und Lungen. Der Polarisator wurde in die Stellung 5° nach links gebracht. Das Signal vom Präparat ergab die Übereinstimmung mit den gleichen Arten von Adenokarzinom.

Das Präparat Doxorubicinum wurde auf die Auflage für die zu untersuchenden Präparate gesetzt. Der Polarisator wurde in die Stellung 5° nach rechts gebracht. Das Signal vom Präparat ergab keine Übereinstimmung mit Adenokarzinomen von Prostata, Mamma, Magen und Lungen.

Der Polarisator wurde in die Stellung 5° nach links gebracht. Das Signal vom Präparat ergab ebenfalls keine Übereinstimmung mit den gleichen Arten von Adenokarzinom.

Um das Vorhandensein oder Nichtvorhandensein von einem malignen Tumor anhand von Heilpräparaten zu bestätigen, wird folgende Vorgehensweise angewendet:

Das Gewebe mit jeweiliger Information wird invertiert und auf einen Informationsträger aufgenommen. Der Informationsträger wird auf dem Objekttisch unter den Polarisator angeordnet und gemäß dem oben beschriebenen Verfahren untersucht. Das invertierte Material des bösartigen Tumors ergibt niedrige Werte in Bezug auf das Bindegewebe und die Isotropie bei der Wendung des Polarisators. Diese Werte nehmen wesentlich zu und werden anisotrop, sobald der Messkreis mit DST-Präparaten ergänzt wird. Das mit keinem Tumor betroffene Gewebe ergibt viel höhere Kennwerte in Bezug auf das Bindegewebe im "0"-Punkt sowie Anisotropie. Diese Werte lassen sich beim Einsatz von DST-Präparaten nicht ändern.

Beispiel 11.

Der Patienten mit Diagnose Hochdruckkrankheit im Stadium 2B. Es wurde der vegetative Resonanztest (VRT) mit der Standardeinrichtung vorgenommen. Die Diagnose wurde bestätigt. Es wurde eine Untersuchung auf das Vorhandensein von malignen Neubildungen bei dem Patienten durchgeführt. Dabei wurde in den Kreis (auf die Auflage für das medikamentöse Testen) ein Fläschchen mit Gentianaviolett eingefügt. Daraufhin erhöhte sich bei dem Patienten die Potenz nach der Skala des Bindegewebes und der Dissymetriegrad. Bei der Einfügung des Gentianavioletts bei einem Patienten ohne Onkologie werden die Kennwerte nach der Skala des Bindegewebes nicht geändert. Zur Bestätigung der Diagnose wurde zunächst der vegetative Resonanztest (VRT) mit Organopräparaten und onkologischen Nosoden durchgeführt. Aufgrund des Testes wurde Vorsteherdrüsenkrebs diagnostiziert. Aufgrund der zusätzlichen Ultraschalluntersuchung und der Biopsie der Vorsteherdrüse wurde das Vorhandensein eines Adenokarzinomes der Vorsteherdrüse festgestellt.

Beispiel 12.

Auf die Auflage zum Testen wurde ein Prüfglas von 1 ml mit einem DST-Präparat (organischer Farbstoff Malachitgrün) gesetzt. Dieses Präparat wird bei der Behandlung des Adenokarzinoms der Bauchspeicheldrüse angewendet. Aufgrund einer einfachen Prüfung ohne Polarisatoren wurde die Nutzbarkeit dieses Präparats für die Behandlung des Bauchspeicheldrüsenkrebses nicht festgestellt. Nachdem das Informationssignal durch die Polarisatoren und den zwischen den Polarisatoren angeordneten Kristall durchgelassen wurde, wurde von dem Gerät für den vegetativen Resonanztest (VRT) eine ausgesprochen hohe Wirksamkeit dieses Farbstoffes zur Behandlung des Adenokarzinoms der Bauchspeicheldrüse und des Dickdarmes festgestellt.

Beispiel 13.

Der Polarisator kann sowohl zur Behandlung des Krebses als auch einer anderen Pathologie angewendet werden. Zu diesem Zweck wird der Polarisator in den Kreis der Bioresonanztherapie (BRT) eingefügt.

Dem Patienten mit einer Diagnose Hautkrebs (Melanom mit Metastasen) wurden 10 Behandlungen der Bioresonanztherapie (BRT) mit dem Polarisator erteilt. Dabei wurde das in den Organismus zurückkommende Informationssignal von dem Kristall und den Polarisatoren invertiert. Es sei erwähnt, dass der Patient auf keine andere medikamentöse bzw. nicht medikamentöse Weise behandelt wurde. Nach den ersten 1 ½ Monaten der Behandlung wurde der Primärherd um 90 % kleiner und es wurden keine Metastasen in der Leber festgestellt. Vor der Behandlung mit Bioresonanztherapie (BRT) und den Polarisatoren wurde bei dem Patienten auf der rechten Hüfte ein assymetrisches und vielfarbiges Melanom mit unregelmäßigen Rändern diagnostiziert. Bei der Ultraschalluntersuchung wurde im rechten Leberlappen ein 2 x 3 cm großer Herd mit unregelmäßigen Rändern und Perifokalödem (Metastasen) entdeckt. Dem Patienten wurden daraufhin 1 Monat lang Behandlungen erteilt. Der Tumor verkleinerte sich bis zu der bisherigen Größe, wurde symmetrisch und von gleichmäßiger Farbe.

Beispiel 14.

Dem Kranke mit der Diagnose chronisch-destruktive Pneumonie wurden 20 Behandlungen mit der Bioresonanztherapie (BRT) mit den das Informationssignal färbenden Polarisatoren erteilt. Während der Behandlung verschwanden Husten und Schmerzen im Brustkorb, Sputum. 3 Monate nach der Behandlung stellte die CT eine ausgesprochen bessere Gewebsstruktur der Lungen und eine Verkleinerung der paraaortalen und paratrachealen Lymphknoten fest.

Demgemäß wurde ein wirksames Verfahren und eine wirksame Einrichtung zur Bestimmung des Grades der Malignität von Zell- und Gewebsstrukturen (-gefügen) und eine Einrichtung zur Bestimmung des Grades der Isotropie (Symmetrie) von biologischem Material (Objekt) erfunden. Darüber hinaus wurde das Inventar von wirksamen Verfahren und Einrichtungen zur Bestimmung des Grades der Malignität von Zell- und Gewebsstrukturen (-gefügen) und zur Bestimmung des Grades der Isotropie (Symmetrie) von biologischem Material (Objekt) ergänzt.

Dabei wurden:

- die Zuverlässigkeit der Daten bei der Bestimmung (des Grades) der Malignität von Zell- und Gewebsstrukturen (-gefügen) und des Grades der Isotropie (Symmetrie) von biologischem Material (Objekt) erhöht,
- die Verfahrenstechnik vereinfacht,
- die für die Untersuchung in Anspruch genommene Zeit verkürzt und
- die Funktionalität für die Diagnosestellung von verschiedenen Pathologien und für die Bestimmung von aussichtsreichen Richtungen der Behandlung ergänzt.

**Patentansprüche**

1. Verfahren zur Bestimmung der Malignität von Zell- und Gewebsstrukturen (-gefügen), zur Untersuchung der Präparate aus biologischen Geweben, **dadurch gekennzeichnet,**
   **dass** die Untersuchung eines beliebigen biologischen Materials (Lebewesens) durch die Bestimmung des Grades der Anisotropie und der Isotropie der Gewebsgefüge (-strukturen) aufgrund eines ausgeprägten Grades der Symmetrie und Dissymetrie von superschwachen elektromagnetischen Schwingungen (Informationssignale) zustande gebracht wird, wobei diese Schwingungen (Informationssignale) bei dem zu untersuchenden biologischen Material im Kreis der Einrichtung für einen vegetativen Resonanztest entstehen, dass die elektromagnetischen Schwingungen durch einen Polarisator durchgelassen werden,
   **dass** die Richtung und der Grad ihrer Polarisation bei der für das Bindegewebe eigenen Resonanzfrequenz als Indikator für den Grad der Malignität angesehen wird und
   **dass** bei der Auswertung berücksichtigt wird, dass das maligne Gewebe durch ein symmetrisches (isotropes) Signal und das Normalgewebe durch ein dissymetrisches (anisotropes) Signal **gekennzeichnet** wird.

2. Verfahren nach Anspruch 1,
   **dadurch gekennzeichnet,**
   **dass** der Index der Symmetrie (Isotropie) und der Dissymmetrie (Anisotropie) nach folgendem Verhältnis berechnet wird:

$$I_{CT} = C \cdot (D : L)$$

dabei sind

I - der Symmetrieindex des Informationssignals
C - das normale Signal des Bindegewebes
D - der Signalpegel mit rechter Polarisation
L - der Signalpegel mit linker Polarisation.

**3.** Verfahren nach Anspruch 1,
**dadurch gekennzeichnet,**
**dass** das zu untersuchende biologische Material durch

- ein histologisches Präparat,
- Vollblut, Blutplasma oder eine andere biologische Flüssigkeit aus der Gruppe: Urin, Speichel, Sperma, Tropfen und / oder
- ein Stück von einem Makropräparat bzw. seinem Abdruck auf einem beliebigen Material auf dem Objektglas repräsentiert wird.

**4.** Verfahren nach Anspruch 1,
**dadurch gekennzeichnet,**
**dass** das zu untersuchende biologische Material durch ein biologisches Subjekt (Mensch, Tier und Ähnliches) gebildet wird.

**5.** Verfahren nach Anspruch 1,
**dadurch gekennzeichnet,**
**dass** das zu untersuchende biologische Material durch eine Information vertreten wird, die diesem Material entnommen und in einem Informationsspeicher aufgenommen wurde (Kapsel, homöopathisches Granulat, Alkohol, Wasser usw.).

**6.** Verfahren nach Anspruch 1,
**dadurch gekennzeichnet,**
**dass** das zu untersuchende Material durch ein beliebiges lebendiges oder nicht lebendiges Material oder eine Substanz gebildet wird.

**7.** Verfahren nach Anspruch 1,
**dadurch gekennzeichnet,**
**dass** der Grad der Malignität des Gewebes mittels Präparate aus der Gruppe Farbstoffe, flüssige Kristalle u. a. Werkstoffen bestimmt wird, wobei diese Werkstoffe Polarisations- bzw. Oszillationseigenschaften aufweisen.

**8.** Verfahren nach Anspruch 1,
**dadurch gekennzeichnet,**
**dass** die Behandlung der onkologischen und/ oder somatischen Erkrankungen mittels Polarisator durchgeführt und der Polarisator dabei in den Kreis der Bioresonanztherapie (BRT) eingefügt wird.

**9.** Einrichtung zur Ausführung des Verfahrens nach einem der Ansprüche 1 - 8, **dadurch gekennzeichnet,**
**dass** sie eine Auflagefläche (eine Schale) für das zu untersuchende Material aufweist,
**dass** diese Auflagefläche unterhalb eines unteren festen Polarisator angeordnet ist,
**dass** über der Auflagefläche und dem festen Polarisator zwei Polarisatoren angeordnet sind, zwischen denen ein Kristall angeordnet ist,
**dass** einer der Polarisatoren fixiert ist und der andere an einem beweglichen Ring mit einer Teilung (Eichung) darauf befestigt ist,
**dass** über dem oberen beweglichen Polarisator sich ein Behälter (eine Schale) befindet, welcher mittels eines Kabels mit der Aufnahmestelle für MT (medikamentöses Testen) des Gerätes für VRT (vegetativen Resonanztest) verbunden ist und
**dass** die Auflagefläche zum Testen und der Behälter aus einem beliebigen Werkstoff ausgebildet sind.

**10.** Einrichtung zur Bestimmung des Grades der (Symmetrie) Isotropie des biologischen Materials,
**dadurch gekennzeichnet,**
**dass** ein Polarisator in Form von zwei Kristallen verwendet ist,
**dass** der eine Kristall über dem unteren unbeweglichen Polarisator angeordnet ist und der zweite Kristall sich unter dem oberen beweglichen Polarisator angeordnet ist und
**dass** sich dieser zusammen mit dem letzteren bewegt.

**11.** Einrichtung nach Anspruch 13,
**dadurch gekennzeichnet,**
**dass** der Polarisator aus magnetischem oder nicht magnetischem Material ausgebildet ist.

12. Einrichtung nach Anspruch 11,
    **dadurch gekennzeichnet,**
    **dass** zwischen den zwei gekreuzten (und/oder parallelen) Polarisatoren ein beliebiger flüssiger oder fester Kristall
    (z. B. Quarz oder Islandspat) beweglich oder unbeweglich angeordnet ist und
    **dass** die Achsen dieses Kristalls parallel (und/oder normal) zum Vektor des Strahlungsstromes verlaufen.

Passive Elektrode

Aktive Elektrode

Patient

Anlage
für vegetativen
Resonanztest
(VRT)

AE
PE
MT

Beweglicher
Kristall

Bildschirm

Umgesetzte
Information

Polarisator

Information
des Patienten

Fig. 1

20

Fig. 2

Fig. 3

c)

b)

a)

Fig. 4

Europäisches
Patentamt

European
Patent Office

Office européen
des brevets

## EUROPÄISCHER TEILRECHERCHENBERICHT

der nach Regel 63 des Europäischen Patentübereinkommens für das weitere Verfahren als
europäischer Recherchenbericht gilt

**Nummer der Anmeldung**

EP 09 00 8368

### EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (IPC) |
|---|---|---|---|
| X | DE 10 2005 005757 A1 (OCTAX MICROSCIENCE GMBH [DE]) 10. August 2006 (2006-08-10) * das ganze Dokument * ----- | 9 | INV. G01J4/00 A61N1/40 G01N21/21 G01D21/00 G01N21/64 |
| X | M.G.TOMILIN: "LC vision application to malignant tumor detecting" THE INTERNATIONAL SOCIETY FOR OPTICAL ENGINEERING, [Online] Bd. 5289, 2004, Seiten 74-85, XP002542698 ISSN: 0277-786X ISBN: 0-8194-5192-4 Gefunden im Internet: URL:http://spied1.aip.org/getpdf/servlet/G etPDFServlet?filetype=pdf&id=PSISDG0052890 00001000074000001&idtype=cvips&prog=normal > [gefunden am 2009-08-18] * das ganze Dokument * ----- -/-- | 9 | |

**RECHERCHIERTE SACHGEBIETE (IPC)**

G01D
A61B
G01N
A61N
G01J
G02B

### UNVOLLSTÄNDIGE RECHERCHE

Die Recherchenabteilung ist der Auffassung, daß ein oder mehrere Ansprüche, den Vorschriften des EPÜ in einem solchen Umfang nicht entspricht bzw. entsprechen, daß sinnvolle Ermittlungen über den Stand der Technik für diese Ansprüche nicht, bzw. nur teilweise, möglich sind.

Vollständig recherchierte Patentansprüche:

Unvollständig recherchierte Patentansprüche:

Nicht recherchierte Patentansprüche:

Grund für die Beschränkung der Recherche:

Siehe Ergänzungsblatt C

| Recherchenort | Abschlußdatum der Recherche | Prüfer |
|---|---|---|
| Den Haag | 25. August 2009 | Crisan, Carmen-Clara |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04E09)

Europäisches
Patentamt

European
Patent Office

Office européen
des brevets

**EUROPÄISCHER
TEILRECHERCHENBERICHT**

Nummer der Anmeldung

EP 09 00 8368

| | **EINSCHLÄGIGE DOKUMENTE** | | **KLASSIFIKATION DER ANMELDUNG** (IPC) |
|---|---|---|---|
| Kategorie | Kennzeichnung des Dokuments mit Angabe, soweit erforderlich der maßgeblichen Teile | Betrifft Anspruch | |
| X | COSTAS BALAS: "A Novel Optical Imaging Method for the Early Detection, Quantitative Grading, and Mapping of Cancerous and Precancerous Lesions of Cervix"<br>IEEE TRANSACTIONS ON BIOMEDICAL ENGINEERING, IEEE SERVICE CENTER, PISCATAWAY, NJ, US,<br>Bd. 48, Nr. 1,<br>1. Januar 2001 (2001-01-01), XP011007010<br>ISSN: 0018-9294<br>* Seiten 97-99 *<br>----- | 9 | |
| X | GB 791 064 A (BRITISH CELANESE)<br>19. Februar 1958 (1958-02-19)<br>* das ganze Dokument *<br>----- | 9 | **RECHERCHIERTE SACHGEBIETE** (IPC) |
| A | US 2003/138378 A1 (HASHIMSHONY DAN [IL])<br>24. Juli 2003 (2003-07-24)<br>* Absätze [0002], [0070] - [0161]; Ansprüche; Abbildungen *<br>----- | 9 | |
| A | EP 0 694 282 A (BRIDGES JACK E [US] INTERSTITIAL LLC [US])<br>31. Januar 1996 (1996-01-31)<br>* Seiten 5-15; Ansprüche; Abbildungen *<br>----- | 9 | |
| A | DE 36 44 705 A1 (KANZAKI PAPER MFG CO LTD [JP] NEW OJI PAPER CO LTD [JP])<br>2. Juli 1987 (1987-07-02)<br>* das ganze Dokument *<br>----- | 9 | |
| A | DE 30 38 325 A1 (MERSMANN LUDGER)<br>29. April 1982 (1982-04-29)<br>* Seite 116 - Seite 126 *<br>* Seite 132 - Seite 133; Ansprüche; Abbildungen *<br>----- | 9 | |

EPO FORM 1503 03.82 (P04C12)

**Europäisches Patentamt**
**European Patent Office**
**Office européen des brevets**

**UNVOLLSTÄNDIGE RECHERCHE**
**ERGÄNZUNGSBLATT C**

**Nummer der Anmeldung**

EP 09 00 8368

Vollständig recherchierte Ansprüche:
9

Unvollständig recherchierte Ansprüche:
10-12

Nicht recherchierte Ansprüche:
1-8

Grund für die Beschränkung der Recherche (nicht patentfähige Erfindung(en)):

Artikel 53 (c) EPÜ - Diagnostizierverfahren, die am menschlichen oder tierischen Körper vorgenommen werden


-----

Weitere Beschränkung der Recherche

Vollständig recherchierte Ansprüche:
9

Unvollständig recherchierte Ansprüche:
10-12

Nicht recherchierte Ansprüche:
1-8

Grund für die Beschränkung der Recherche:

Die vorliegende Anmeldung enthält 12 Ansprüche, von denen 3 unabhängig sind. Es gibt keine deutliche Unterscheidung zwischen den unabhängigen Ansprüchen 9 und 10, weil sie im Schutzbereich miteinander überlappen. Sie sind so gefaßt, dass diese Ansprüche insgesamt die Erfordernisse des Artikels 84 EPÜ hinsichtlich der Klarheit und der Knappheit nicht erfüllen, da es für den Fachmann besonders aufwändig ist, den Gegenstand festzustellen, für den Schutz begehrt wird. Die Verletzung der einschlägigen Erfordernisse ist so schwerwiegend, dass eine sinnvolle Recherche des ganzen beanspruchten Gegenstandes nicht durchgeführt werden konnte (Regel 63 EPÜ und Richtlinien B-VIII, 3). Der Umfang der Recherche wurde deshalb begrenzt.

Die Recherche wurde für den Gegenstand durchgeführt, von dem, soweit verständlich, vernünftigerweise erwartet werden konnte, dass er später im Verfahren beansprucht wird, nämlich der entsprechende Anspruch 9.

**ANHANG ZUM EUROPÄISCHEN RECHERCHENBERICHT
ÜBER DIE EUROPÄISCHE PATENTANMELDUNG NR.**

EP 09 00 8368

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-08-2009

| Im Recherchenbericht angeführtes Patentdokument | Datum der Veröffentlichung | Mitglied(er) der Patentfamilie | | Datum der Veröffentlichung |
|---|---|---|---|---|
| DE 102005005757 A1 | 10-08-2006 | WO | 2006081791 A1 | 10-08-2006 |
| | | EP | 1846739 A1 | 24-10-2007 |
| | | US | 2008170227 A1 | 17-07-2008 |
| GB 791064 A | 19-02-1958 | KEINE | | |
| US 2003138378 A1 | 24-07-2003 | US | 2009187109 A1 | 23-07-2009 |
| | | US | 2007260156 A1 | 08-11-2007 |
| EP 0694282 A | 31-01-1996 | DE | 69532367 D1 | 05-02-2004 |
| | | DE | 69532367 T2 | 21-10-2004 |
| | | US | 6061589 A | 09-05-2000 |
| DE 3644705 A1 | 02-07-1987 | JP | 1791485 C | 14-10-1993 |
| | | JP | 4078137 B | 10-12-1992 |
| | | JP | 62157549 A | 13-07-1987 |
| | | US | 4849623 A | 18-07-1989 |
| DE 3038325 A1 | 29-04-1982 | KEINE | | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- RU 2140661

**In der Beschreibung aufgeführte Nicht-Patentliteratur**

- **M.V. Kutushov.** Krebs ist heilbar. 2005, 86-87
- **Sivukhin D.V.** Allgemeine Physik. Optik. 1985
- **Landsberg G.S.** Optik. 1976
- Rossijskaya Enzyklopedia. 1999, vol. S.90, 460